# EUROPEAN PATENT APPLICATION

(11) **EP 0 725 141 A1**
(43) Date of publication of application: **07.08.1996**
(21) Application number: 94930374.7
(22) Date of filing: 17.10.1994
(51) Int. Cl.: C12N 15/87

(54) **METHOD OF CONTROLLED GENETIC TRANSFORMATION OF AN ANIMAL MAMMARY GLAND AND A DEVICE FOR INTRODUCING GENETIC MATERIAL INTO THE MAMMARY DUCT OF AN ANIMAL MAMMARY GLAND**

(30) Priority: 18.10.1993 RU 93048499
(71) Applicant: Tovarischestvo S Organichennoi Otvetstvennostju "Bioprogress", Moscow 113461 (RU)
(72) Inventor: SOBOLEV, Alexandr Sergeevich, Moscow, 117192 (RU); ROZENKRANTS, Andrei Alexandrovich, Moscow, 117133 (RU); NIKITIN, Vladimir Afanasievich, Puschino, 142292 (RU)
(74) Representative: Einsel, Martin, Dipl.-Phys.
(86) International application number: RU9400235
(87) International publication number: WO9511962

(57) **Abstract**

The proposed method of controlled genetic transformation of a mammary gland involves: the introduction of genetic material into the animal's body in a soluble complex with carriers; the controlled delivery of genetic material by the said carriers to the target cell by specific receptor-mediated endocytosis, and whence to the cell nucleus by cytoplasm-nucleus transport. The said soluble complex is introduced into the animal's body via the mammary duct of the mammary gland. The proposed device for introducing genetic material into the mammary duct of an animal's mammary gland comprises a housing (1) with a depression (2) for accommodating the teat of the mammary gland, a central aperture (3) and an annular channel (4) at the base of the depression (2) which can communicate with a vacuum source for widening the teat sphincter. The central aperture (3) is used to introduce the device (6) through which the genetic material is introduced into the mammary duct.

## Description

### FIELD OF THE INVENTION

The invention relates to animal gene engineering, an experimental and molecular biology, biotechnology and, more particularly, the invention relates to a method for the specific genetic transformation of mammary gland in animal and a device for introducing of genetic material into animal mammary duct.

### BACKGROUND OF THE INVENTION

Nowadays, there are known numerous techniques for genetic modification of cells (O.K.Glebov. Genetic transformation of cells. Leningrad, "Nauka", 1989, p.10,11,16,17).

Most of the known methods are concerned with either transformation of cells grown in culture or breeding of transgenic animals. Thus, for example, the possibility of producing species-different proteins in a milk has been demonstrated in experiments on transgenic animals (Genetic Technology News. 1988, v.7, 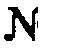 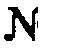 º 9, p.1).

However, only a few of the genetic transformation methods are suited for gene transfer into somatic cells . The suitable methods for genetic transformation by gene transfer to somatic cells in vivo are the techniques using liposomes, viruses with genome into which the required genetic information is inserted and DNA-carrying constructs, as well. The use of soluble DNA-carrying complexes is of the greatest promise for the genetic transformation of somatic cells in vivo.

Thus, for example, EP No.0388758 describes the methods for the genetic transformation in vitro using conjugated carriers, transferrinpolylysine, antibody-polylysine conjugate and the like. Such methods use as the carrier a conjugate comprising a polyatomic cation and a ligand which provides the binding with cells; this conjugate together with a genetic material, nucleic acids, forms a soluble complex; the uptake of such complex by cells in vitro occurs through receptor-mediated endocytosis.

However, the above-mentioned methods are unsuitable for the mamary gland genetic transformation due to its drastically low transfection efficiency (< 0.1%). Thus, the method for the genetic transformation of mammary gland with using conjugates such as transferrinpolylysine is difficult for practical implementation . Furthermore, this method has not been developed for the in vivo introduction; the carriers are prepared together with ligands which fall to be detected in cell nuclei after uptaking them through receptor- mediated endocytosis, and the effective genetic transformation of cells grown in vitro with such carriers is achieved by using other techniques (Cotten M., Wagner E., Zatloukal K., Phillips S., Curiel D.T., Birnstiel M.L. High-efficiency receptor-mediated delivery of small and large (48 kD) gene construct using the endosome-disruption activity of defective or chemically inactivated adenovirus particles. Proc. Natl.Acad.Sci., v.89, p 6094-6098, 1992; Wagner E., Zatloucal K., Cotten M., Kirlappos H., Mechtler K., Curiel D.T., Birnstiel M.L. Coupling of adenovirus to transferrinpolylysine-DNA complexes greately enhances receptor-mediated gene delivery and expression of transfected genes.Proc. Natl.Acad.Sci., v.89, p 6094-6098,1992).

Furthermore, there is known in the art a method for the genetic transformation method, as disclosed in PCT US 92/19749. In this method, the introduction of genetic material into animal body is accomplished in a soluble complex with carriers which deliver the genetic material directly to the target cell by receptor - mediated endocytosis. As it takes place, the targeted gene encoding for cell surface receptors is delivered into animal body in a soluble complex comprising a genetic material and a carrier having a gene-bindings site and a specific cell-recognizing site, with the targeted gene injected intravenously into animal body.

In the above method, a soluble complex, when injected intravenously, is interacted with many of the body's tissues and liquids, that , on the one hand, may result in the increased biological degradation of such water-soluble complex and, on the other hand, can cause the various side-effects in tissues. In addition, large amounts of a genetic material are required to obtain an efficient concentration of such water-soluble complex.

To accomplish the introduction of a genetic material into body cells with using a soluble complex comprising a genetic material and a carrier, there is an urgent need of providing a device which permits one to deliver mostly effectively a genetic material into somatic cells in vivo, in particular, by injecting it through the mammary duct.

In SU patent application, No. 1123598, IPC A01J 5/06, there is disclosed a device, for example, a teat cup, which is intended for sucking a liquid from mammary gland. The device has a case with a recess in the form of animal mammary gland to make the mammary teat secure relative to a central discharge opening which is cut in the bottom of the recess and communicates with a vacuum source.

In order to increase the suction efficiency, the recess has an annular collar that surrounds the central discharge opening and provides the expansion of the teat sphincter (the outlet of mammary duct).

The device operates as follows. An animal teat is placed into the recess of the teat cup such that there is a clearance between the top of the teat and a flat bottom of the recess. Inside the recess, there is generated a negative pressure (vacuum) , and the device is located at the teat bottom. Beneath the teat, there is built up a vacuum space, the teat extends as far as it will go to the annular collar, and the sphincter openes. Once a variable vacuum is generated at the central discharge opening, there occur the suction of the liquid from the mammary gland.

This device, however, makes it impossible to inject a liquid into the mammary duct from the outside, since the teat sphincter expands while the suction of the liquid from the mammary gland occurs. Furthermore, the expansion of the teat sphincter is accompanied with a significant deformation of the teat walls. As it takes place, the ratio of the teat deformation to be required for disclosing the sphincter is determined by the sizes of a recess and animal teat, and when the difference in sees is significant, the teat deformation may cause physical perturbations in the teat tissue and feeling of pain in animal.

In FR-A-2195910, there is disclosed a device for injecting solutions , for example, medicinal agents and/or lubricants, into animal mammary duct, comprising a sphincter dilator having flexible members which, when jointed, form a tip, and a stop that limits the insertion of the tip into the teat.

The stop may have a drain channel, and the flexible members of the dilator may serve as the container for solutions to be injected into mammary duct.

The flexible members is deformable such that they contract while the dilator enters the mammary duct, and expand after insertion to occupy the space of the duct, thereby allowing the device to use in animal with teats different in size.

Should the device be used , the insertion of a dilator into a mammary duct would cause inevitably the deformation not only the teat tissue, but the main duct of mammary gland, as well. Moreover, the stop allows the dilator enter the teat in a given depth only irrespective the teat difference in see in the same animal, as it takes place, for example, in mice.

Furthermore, this device is designed for the insertion of a dilator into the teat sphincter manually without fixation and orientation of it with respect to the mammary gland, that makes it suitable for large animals only, as a teat sphincter is not easy to detect in small mammalian having, for example, a teat of 1-3 mm in diameter and, hence, the insertion of a dilator thereinto is difficult to perform.

### DISCLOSURE OF THE INVENTION

The present invention has as its objects the provision of a method for the specific genetic transformation of animal mammary gland, which could improve its reliability and safety due to producing locally a high concentration of a soluble complex to be used for exogenous gene transfer, thus leading to prevention of undesirable side-effects in tissue and reduction of the added genetic material, and a device for realizing the method.

According to the invention, there is provided a method for the specific genetic transformation of animal mammary gland, comprising the introduction of genetic material into animal body in a soluble complex with carriers which provides the transport of a genetic material directly into cell through the receptor-mediated endocytosis, wherein the genetic material is further introduced into a cell nuclei by cytoplasm-nuclear transport with using the carriers transporting the genetic material directly into the cell nuclei, with the soluble complex of the genetic material with the carriers injected into either cell itself or its nuclei through a mammary duct.

A genetic material is a nucleic acid. According to the present invention, a nucleic acid is DNA. The cell membrane is the difficult-to-penetrate barrier for large hydrophilic molecules as are DNA molecules. For this barrier to be surmounted, it is appropriate to use as the carrier a conjugate of polyatomic cation(s) - ligand(s).

It is appropriate to use a conjugate containing a polyatomic cation which is covalently attached to ligands. In order to provide the reversible binding the carrier with the nucleic acids, polylisine is preferred to use as the polyatomic cation.

A preferable ligand is insulin which, besides the delivery of the genetic material in a water-soluble molecular complex into the cell through the receptor-mediated endocytosis, provides the exogenous gene transfer to the cell nuclei by cytoplasm-nuclear transport. A carrier preferably is the conjugate of polylisine covalently attached to insulin, which is prepared by binding insulin covalently to polylisine. To this end, the terminal insulin ends is modified with citraconic arhydride. Then, polylysine and insulin are treated with succinimidylpyridinedithiopropionate. The pyridindithionylated polylysine is reduced. Both components are dissolved in 2 M guanidine. The pyridindithionylated insulin is treated with freshly reduced SH-polylisine to obtain with 10% a modification of amino groups by titration under stirring and the resulting product is incubated.

The method of the present invention allows one to accomplish the in vivo specific genetic modification the mammary cells. In the method of the present invention, the use of a special-purpose carrier provides the specific transport of a genetic material included in the artificial water-soluble molecular complex both into the cell through the receptor-mediated endocytosis and into its nuclei by cytoplasm-nuclear transport. The injection of the water-soluble carrier-gene material complex into a mammary duct allows one to produce in situ the equivalent concentration of the complex by using smaller amounts of the material as compared with the known method wherein a genetic material is infused intravenously . All the above-mentioned advantages provide the high reliability, safety and efficiency of the method of the present invention.

For the embodiment of the proposed method for the specific genetic transformation of mammary gland, the inventions provides as its object a device for injecting the genetic material into animal mammary duct, which is a conventional teat cup per se, comprising a case with recess for placing the teat of mammary gland with tightly-fitting therein, and a central opening cut at the bottom of the recess, with the recess having an annular groove that surrounds the central opening and communicates with the vacuum source that provides the teat sphincter dilation, and the central opening serving as the entry for the transport means that provides the introduction of a genetic material into the mammary duct.

For the device to be unified, the case may be manufactured from a plastic material and provided with the means for changing the working volume and the of the recess. The transport means may be a conventional cannula per se, which comprises a measuring capacity for a genetic material, and the hollow tip with its outer diameter being equal to the diameter of the central opening, through which the genetic material is injected into a mammary duct.

The measuring capacity may be manufactured from a plastic material and may serve, on the one hand, as the space for the placement of a genetic material, and on the other hand as the means to inject such material into the tip.

In order to prevent injuries, when the device is under operation, and the increase of increase the accuracy of injecting the material in, the tip may have a needle being tapered at 30-45° longitudinally to the tip, and the needle edges may be fused.

To provide the closest contact of the tip with the sphincter and the saving of a genetic material, the outer side of the tip may be conical in shape.

Such design of the device makes it possible to increase the reliability under operation, the safety and the efficiency of injection of a genetic material into a mammary duct due to providing the lower deformation of animal teat, the introduction of a solution into the duct, no matter what the size of the teat and the strict dosage of the injected solution.

### BRIEF DESCRIPTION OF THE DRAWINGS

For a better understanding of the present invention, some specific examples illustrating its embodiments are given below.
Fig. . 1 - A schematic view of a device for injection of genetic material into animal mammary duct;
Fig.2 - A graphical representation of purification process of insulin-polylisine carrier, accomplished by gel-filtration on sephacryl 200 column;
Fig.3 - The solubility of carrier-genetic material complex (insulin-polylisine-plasmid) versus the molar ratio of lysine to nucleotide therein;
Fig.4 - The content of low-density and high-density fractions of plasmid DNA in insulin-polylisine-plasmid complexes versus the molar ratio of lysine to nucleotide therein under analytical ultracentrifugation;
Fig.5 - The sedimentation constants of light and heavy components of low-density fraction of plasmid DNA in insulinpolylysine/plasmid DNA complex versus the molar ratio of lysine to nucleotide in soluble complex under analytical centrifugation;
Fig.6 - The displacement cell hepatome PLC/PRF/5 specific-bound [¹²⁵I]by soluble carrier-genetic material complex(insulin-polylisine-plasmid pSVRP8), and by unlabelled insulin;
Fig.7- The binding of soluble carrier-genetic material complex with cell PLC/PRF5 line; FITC-labelled insulin-polylisine-DNA complex on cell surface,as measured by confocal laser scanning microscopy;
Fig.8 - The receptor - mediated endocytosis of soluble carrier - genetic material complex in cell PLC/PRF5 line;
   accumulation FITC-labelled of insulin-polylysine-DNA complex in cytoplasm,as measured confocal laser scanning microscopy;
Fig.9 - The kinetics of transport of conjugated carrier, insulinpolylysine into cell PLC/PRF5 nuclei;
Fig. 10 - The cytoplasm-nuclear transport of soluble carrier-genetic material complex in cell PLC/PRF5 line; accumulation fluorescene-labelled insulin-polylisine-DNA complex, as measured confocal laser scanning microscopy;
Fig.11 - The specific genetic transformation of cell grown in culture, estimated from the expression efficiency of major SV 40 virus T-antigen in human cell hepatome PLC/PRF5 line, as transformed by insulin-polylisine-plasmid complex;
Fig. 12 - The specific genetic transformation of mamary secretory cells grown in culture of Lampyrida luciferase genome by the direct delivery insulin-polylisine-plasmid DNA pRSVl;
Fig. 13 - The in vivo genetic transformation of mice mammary cells.

Preferred embodiment of the invention.

According the invention, there is provided a method for the in vivo specific transformation of mammary cells. A cell membrane is a difficult-to-surmount barrier for large hydrophilic molecules, which are DNA molecules.

To surmount this barrier, there is used a specific carrier comprising a ligand (s) and a polyatomic cation(s). A cell-internalized receptor-specific ligand may transport a genetic material as a component of an artificial soluble molecular complex directly through the outer cell membrane. The nuclear membrane is the second barrier which should be surmounted for the effective genetic transformation of nonproliferating interphase cells. The use of the ligand that provides the entry of a genetic material into nuclei, as a component of such molecular construct, permits one to overcome this problem.

The use of a carrier that is able of providing the cytoplasm-nuclear transport, makes it possible to deliver a genetic material directly to that cell compartment, where processing of the genetic information occurs. It is entirely possible that injection of such soluble carrier - genetic material complex into a mammary duct, permits one to obtain locally the same concentration of the complex due to smaller amounts of the material used as compared with with intravenous injection it is infused intravenously.

The specificity and the efficiency of the method is accomplished by the combination of several techniques including the local introduction of a solution containing such complex with a specially designed device, the use of an artificial soluble molecular construct of carrier ligand(s)-polyiatomic cation (s) with DNA, wherein one may use just as the ligand ligand that provides the cell specificity and the transport of an artificial molecular construct inside the cell through the receptor - mediated endocytosis, so the ligand that provides the entrance into cell nuclei through cytoplasm-nuclear transport.

In this case, the cytoplasm-nuclear transport mechanism used for the delivery of gene construct directly to cell nuclei can be accomplished through the binding of the ligand added to the construct with any cell component which provides its entry to the cell nuclei. A suitable ligand is also a ligand which provides both the delivery of the construct into target cell through receptor - mediated a endocytosis and the delivery of the construct into the cell nuclei by cytoplasm-nuclear transport. It is well suited to use as such ligands, for example, insulin, prolactin, epidermal growth factor of growth and the like ( Burwen S.E., Jones F.L. The accumulation of polypeptide hormones and growth factors with the nuclei of target cells. Trends Biochem. Sci., v.12, p.159-162 (1987)8 Clevenger C.V., Sillman A.L., Prystowsky M.B. (1990) Interleukin-2-driven nuclear translocation of prolactin in cloned T-lymphocytes. Endocrynology, 127, 6 , 3151-3159 (1990)).

In case the ligands of two types are used, namely, a ligand that provides receptor-mediated endocytosis and ligand for the cytoplasm- nuclear transport, it is possible to use as the ligand, for example, somatotropin, transferrin and the like. ligand. In this case, it is appropriate to use also a ligand that provides the cytoplasm-nuclear transport. Such ligands can include, for example the peptides with nucleolisation signals. ( Goldfarb D.S. Gariepy J., Schoolnik G., Kornberg R.D. Synthetic peptides as nucalisation signals. Nature, 322, 641-644 (1986)), engineered proteins with signals nucleoplasmin and the like ( Rihs H.-P., Peters R. Nuclear transport kinetics depends on phosphorylation-site containing sequences flanking the karyophilic signal of the Simian virus 40 T-anigen. EMBO J.8, 1479-1484 (1989); Garcia-Bustos J., Heitman J., Hall M.N. Nuclear protein localization. Biochem. Biophys. Acta 1071, 83-1-1 (1991). A suitable ligand as a carrier of an artificial, soluble, molecular construct to the cell nuclear for the in vivo transformation is insulin. It is a well common knowledge that there are receptors on a surface of different cell types; these receptors binds insulin and in a complex with hormone are absorbed by a cell by receptor - mediated endocytosis ( Smythe E., Warren G. The mechanism of receptor-mediated endocytosis. Eur.J.Bioch., v.202. p. 689-699(1991). One of the compartments where insulin accumulates following the receptor-mediated endocytosis is a cell nuclei (Smith R. M., Jarret L. Partial characterization of mechanism of insulin accumulation in H35 hepatoma cell nuclei. Diabets, v.39, p.638-689 (1990)).

The use of an insulin for genetic transformation of mammary cells is quite justified, as far as the cells of this tissue have insulin receptors. For specific genetic transformation of mammary cells, the most promising technique is the local introduction of artificial water-soluble molecular construct because smaller amounts of the material are used, and its high concentration is reached locally. Its it essential that the first cell type, according to the invention, with which the injected substances interact, are mammary epithelial secretory cells that are of particular interest biotechnologically. A suitable polyatomic cation that provides reversible binding the carrier with nucleic acids is, for example, polylysine, synthetic polyatomic cations, cationized proteins and the like.

Based on the foregoing, a carrier for a genetic material, according to the invention, is insulin as covalently attached to polylysine ( insylin-polylisine conjugate). The synthesis of the insulinated polylysine is as follows: insulin is first labeled with ¹²⁵ I with subsequent citraconylation of it, and polylysine is firstly labeled with [¹⁴ C-] dinitrofluorobenzene or ³⁵S-buthoxycarbonylmethyonine, the resulting components, prior to the cross-lining, is treated in 2 M guanidine, the pyridindithionylated insulin is titrated polylysine up to 10% a modification of amino groups, the cross-linking of insulin with polylysine is carried out with succinimidylpyridinedithiopropionate, the resulting product is chromatographed with subsequent removal of citraconyl groups from insulinpolylylisine.

A method for the specific genetic transformation of animal mammary gland using the proposed device for the injection a genetic material to mammary duct is accomplished as follows. The insunilated polylysine used as carrier for genetic material is prepared as described above. The synthesized carrier can be stored in a frozen state at temperature - 20 within several months. The resulting complex is transferred into Hanks' solution, pH 7.4. The complex as formed in Hanks' solution is charged into a device for the section of a genetic material into a mammary duct. Such complex solution is injected into animal mammary duct using a device of the present invention.

According to the invention, a device has case 1 with recess 2 to place the teat of a mammary gland with tightly- fitting therein, and central opening 3 at the bottom of recess 2. At the bottom of recess 2, there is annular groove 4 that surrounds the central opening 3 and communicates with the vacuum source 5, and central opening 3 serves as the entry for transport means 6 that provides the introduction of a genetic material.

Case 3 can be made from a plastic material and designed with the means to change the working volume and the shape of the recess, that makes the device being adapted for animals having different mammary teats in sizes.

Transport means 6 is a conventional cannula per se, having measuring capacity 7 for a genetic material, and hollow tip 8 to supply the added material to animal mammary duct, with its outer diameter being equal to that of central opening 3.

Measuring capacity 7 may be manufactured from a plastic material and may serve, on the one hand, as the vessel for a genetic material, and on the other hand as the means to intoduce such material into tip 8 .

Tip 8 has needle 9 being tapered at 30-45^{°} longitudinally to tip 8, and edges of needle 9 are fused to increase the efficiency and prevent the mammary gland from injuries under operation of the device.

To save a genetic material, the outer side of tip 8 may be conical shape for providing the closure of central opening 3 by transport means 6, while the material enter the mammary duct.

The device operates as follows. Into recess 2 of case 1, there is placed a mammary teat with pressing case 1 to animal body. Then , vacuum is built up in annular groove 4, for example, through a channel 5. The teat surface, adjacent to annular groove 4, is deformed followed by its expansion at the sphincter, the sphincter openes with being fixed in central position to central opening 3.

First, into measuring capacity 7 of transport means 6, a strict amount of a genetic material is charged. Tip of 8 of transport means 6 is inserted through central opening 3 into the teat sphincter. Then, the material is supplied from measuring capacity 7 through hollow tip 8, for example, by displacing or injecting , into mammary duct.

To increase the contact between the device and the surface of the mammary teat and safety of the device under operation, recess 2, annular groove 4 and central opening 3 is preferably filled or wetted, with a liquid, for example, they may be filled with isotonic solution.

After injection of the genetic material, the negative pressure is released from annular groove 4, transport means 6 is removed from central opening 3, the sphincter closes, the teat returns to normal, unreformed state, and the device is removed .

Under operation of the device, the suck of the teat across annular groove 4 causes the teat fixation and the sphincter expansion, when the teal is deformed partially, thereby preventing the considerable deformation, both teat tissue and main mammary duct, while tip 8 of transport means 6 is inserted. With needle 9 of tip 8 being tapered at a angle of 30-45° and its edges being fused, the device of the invention prevents epithelial tissue of the mammary duct from injuries.

Thus, the device of the present invention makes it possible to simplify and cut considerably the injection of liquids to a mammary duct, thereby allowing to provide a high safe-keeping of a biological object and the all-purpose application for numerous mammalian species, independently on animal condition, without using auxiliary means.

For a better understanding of the present invention, some specific examples illustrating its embodiment are given hereinbelow.

### EXAMPLE 1

### Synthesis and purification of the insulinated polylysine as the carrier for a genetic material

Prior to the carrier preparation, bovine insulin (Sigma) was iodinated with ¹²⁵I using iodogene in buffer 1 (50 mM HEPES (Sigma), pH 7.5 at 20^{°}C, 150 m NaCl). ¹²⁵I-labeled insulin was purified by the Sephadex G 25 gel-chromatography in buffer 1. The radioactivity of the resulting preparation was measured by the gamma-ray counter, Rigamma 1271 (LKB, Sweden).

Prior to the carrier preparation, poly-L-lysine (90 kDa) (Sigma) was labeled with ¹⁴C⁻ dinitrobenzene (Amersham). The labeling was conducted in 0.5 M Na-phosphate buffer at pH 9.5. The labeled polylysine was purified by ultrafiltration against buffer 1. The radioactivity of the resulting preparations was measured by the liquid scintillation counter, Rackbeta 1271 (LKB, Sweden).

Bovine [¹²⁵I] insulin was covalently attached to ¹⁴C-labeled polylysine with a bifunctional cross-linking reagent, N-succinimidyl-3-(2-pyridyldithio)-propionate (SPDP) (Sigma) according to the procedure disclosed by Jung et al (Jung G., Kohnlein W., Lauders G., Biological activity of the antitumor protein neocarcinostatin coupled to a monoclonal antibody by N-succinimidyl-3-(2-pyridyldithio)-propionate. Biochem. Biohys.Res.Commun., v.101, p.599-606 (1981)).

Prior to modification, the N-terminal insulin amino groups were protected with citraconic anhydride (Serva) in a manner as disclosed by Rosenkranz et al. (Rosenkrants A.A., Yachmenev S.V., Jans D.A., Serebryakova N.V., Murav'ev V.I., Peters R., Sobolev A.S. Receptor-mediated endocytosis and nuclear transport of a transfecting DNA contruct. Exp.Cell Res., v.199, p.323-329 (1992)). To 20 ml (40µg) of the bovine radiolabeled insulin in 25 mM BES, pH 6.9 at 20⁰C, there were added 6.6 µ l of citraconic anhydride under stirring continuously. After 1 h of the incubation at 20⁰C with adjusting pH to 6.9, insulin was dialysed against buffer 1. Thereafter, 500 µl of 40 mM SPDP in methanol were added to insulin. After 30 min. of the incubation at 23⁰C, the sample was also subjected to ultrafiltration against buffer 1. The degree of the insulin modification was examined spectrometrically by the occurrence of the reduction product, pyridine-2-thione.

Polylysine was modified with SH-groups using SPDP. To 1050 µl (28.3 mg) of the radiolabeled polylysine in buffer 1, 326 µl of 40 mM SPDP in methanol were added. After 45 min. of the incubation at 25⁰C, the modified polylysine was subjected to the ultraliltrarion against buffer 1. After dialysis, dithiopyridinyl polylysine groups were reduced to SH-groups by the addition of dithiothreitol at the final concentration of 50 mM. After 30 min. of the incubation at 37^{°}C, the resulting product was subjected to the ultraliltration against buffer 1. The degree of the polylysine modification with SPDP was determined prior to dialysis by the same procedure as disclosed above for insulin. The SH-group content in polylysine was of 10 % under conditions as disclosed.

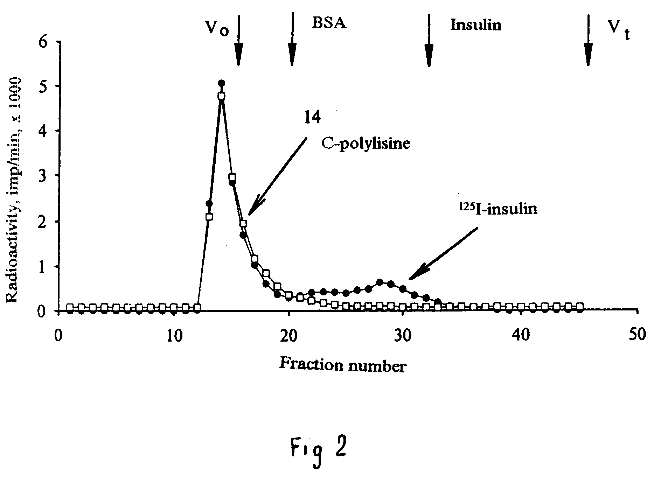

Prior to cross-linking, both insulin and polylysine were placed in buffer 2 ( 50 mM HEPES, pH 7.5, 20^{°}C, 150 mM NaCl, 2 M guanidine (Merck)). To prepare the conjugate, 1365 µl (9.6 mg) of SH-polylysine were dropwise added to 6280 µl (9.7 mg) of the pyridindithionylated insulin under stirring vigorously. The conjugation was conducted in buffer 2 for 18 h at 4^{o}C. The reaction of the dithiopyridynyl insulin groups with SH-groups derived after reducing the dithiopyridynyl polylysine groups was examined spectrometrically, as disclosed above. The resulting conjugate was purified by the sephacryl S-200 gel-chromatography (Pharmacia) in buffer 2, as shown in Fig.2 which illustrates the elution profile of the conjugated [ ¹²⁵I ]- insulin[¹⁴C ]-polylysine, and wherein Vₒ- the free column space ; Vₜ -the total column volume; BSA-the peak of bovine serum albumin elution; insulin - the peak of insulin elution. The citraconyl groups of insulin ( included in the conjugate) were deprotected, as disclosed by Rozenkrants et al. To the purified conjugate in buffer 2, 1 M HCl was added, with adjust pH to 2.0. After 4 h of the incubation at 18^{°}C ( under maintaining pH at 2.0), the solution was sharply adjusted to 9.3 by addition of 1 M NaOH. Thereafter, the resulting insulin-polylisine was three-fold dialyzed against 0.15 M Na Cl and concentrated by ultrafiltration. The molar ratio of insulin and polylysine was estimated by the ¹²⁵I- ¹⁴C ratio in the conjugate, which was of from 8 to 10 insulin residues per 1 polylysyne residue in various series of the synthesized product.

Such method provides the effective insulin-polylysine coupling as evidenced by the results of the conjugate gel-filtration, as shown in Fig. 2. The labeled insulin and the labeled polylysine were eluted from the columns as the same fractions. Up to 80% of insulin participating totally in the reaction were coupled with polylysine, and the yield of the resulting product was of 50%.

### EXAMPLE 2

### Preparation of soluble DNA complex with a carrier

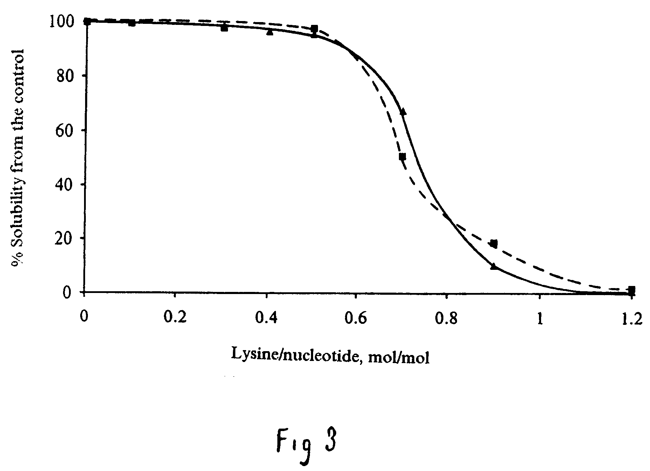

The solubility of various DNA complexes with a carrier (insulin-polylisine-plasmid DNA) against the molar ratio of lysine to nucleotide in the complex was shown, as the curve, in Fig.3. The solubility of insulin-polylisine-DNA complex was substantially independent on the type and the size of plasmid DNA included in the complex. Thus the complexes of DNA constructed with the plasmid, pSVRP8 ( 5.9 kbp ), and pSV3neo ( 8.6 kbp), designated in Fig.3, correspondingly, as squares and e triangles, were soluble up to the molar lysine/nucleotide ratio of 0.5 The production of insulin-polylisine-plasmid DNA complexes were examined by the agarose-gel electrophoresis and analytical ultracentrifugation.

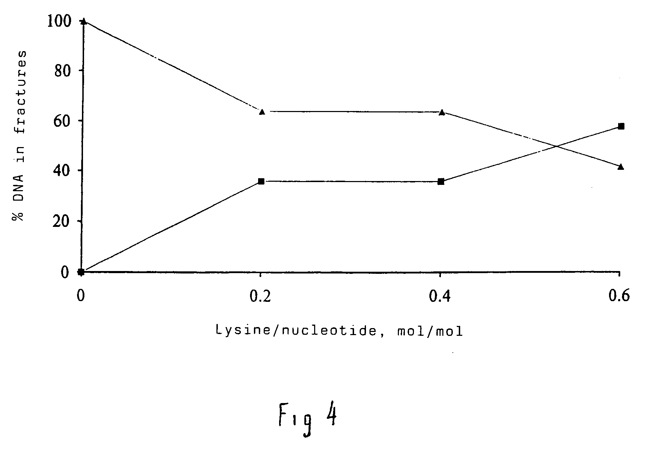

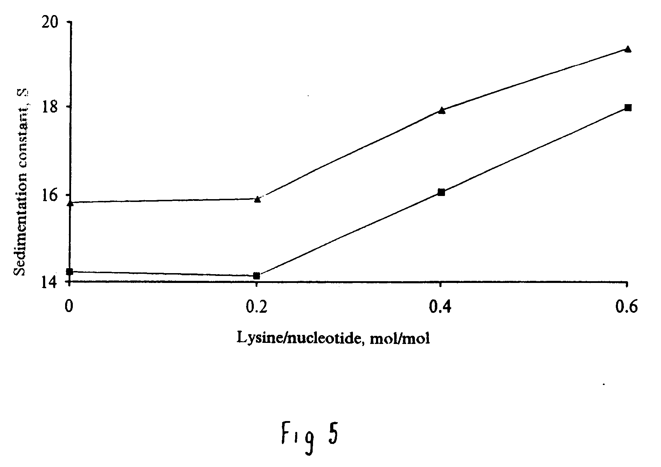

The results of a electrophoresis ( 1 % agarose gel) indicates that the content of a plasmid DNA as a component of insulin-polylisine- plasmid pSVRP8 complex, remaining at the start as stable DNA fraction, grew with increasing the lysine/nucleotide ratio (0.1-0.5) . The similar results were obtained under analytical ultracentrifugation of soluble insulin-polylisine-plasmid pSVneo complex with different molar ratio between lysine and nucleotide monomers ( Fig. 4 and 5).

As seen from the sedimentation profile, the complexes contained a low-density fraction of DNA ( designated in Fig. 4 as triangles ) ( consisting from the two components ) with variable sedimentation constant 14-19 S ( depending on lysine / nucleotide ratio), and a high-density fraction of DNA ( designated in Fig. 4 as squares ) ( soluble microaggregates ), which were participated by ultracentrifugation on the tube bottom.

With increase of lysine/nucleotide ratio ( Fig. 4), the content of low-density fraction decreased and, correspondingly, the content of high-density fraction of plasmid DNA grew in such soluble complex.

In turn, the increase of lysine / nucleotide ratio (0.2-0.6) resulted in the increase of sedimentation constants of light and heavy components of low-density fraction of plasmid DNA, as shown in a Fig. 5, where the squares mean sedimentation constant of light component; triangles - sedimentation constant of heavy component; S- Swedberg units.

The above data allows one to conclude that the carrier of the invention is able of producing soluble complexes with a genetic material, namely, plasmid DNA.

### EXAMPLE 3

### Study of receptor-binding properties of a soluble DNA complex with carrier

PLC / PRF / 5 cells , as our results indicate, have one type of receptors binding insulin, with dissociation constant of complex containing receptor- insulin - 3.7 nM, and their content per cell, (16 x 10⁴, that make such receptors convenient as the object for study of receptor - binding properties of soluble a carrier- genetic material complex (insulin-polylisinea plasmid ). The affinity of insulin-polylisine / plasmid pSVRP8 complexes to insulin receptor of cell PLC / PRF / 5 were determined by competitive ligand-binding radioimmune analysis. Cell were cultivated in RPMI-1640 medium supplemented with 10 % fetal calf serum.

To determine the extent of binding, cell were incubated for 18 hours at 0^{o}C with ¹²⁵I - labeled insulin ( at concentration 0.3-0.5 nM), at growing concentration of the complexes ( here and thereafter the concentration given based on total DNA ), or with unlabeled insulin.

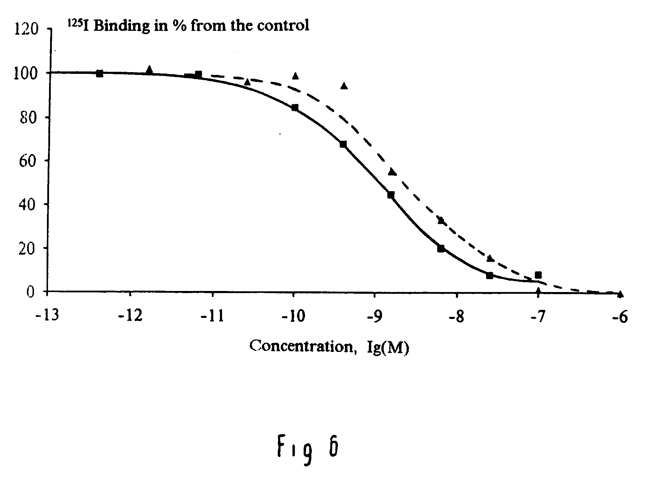

The soluble complexes insulin-polylisine/ plasmid (lysine-nucleotide ratio - 0.4 ) were competed with effectively with radio-labeled ligand for cell hepatome insulin receptors with the identical affinity as that of unlabeled insulin, since displacement curves in both cases were substantially coincided, as shown in a Fig.6 where the squares mean a carrier - genetic material complex, and triangles - unlabeled insulin. The similar results were obtained also for complexes of containing a carrier with other plasmid pSV3 neo.

The obtained results indicates that insulin, as a component of soluble insulin-polylisine- plasmid DNA complex, remains the ability of binding the insulin receptors on cell surface.

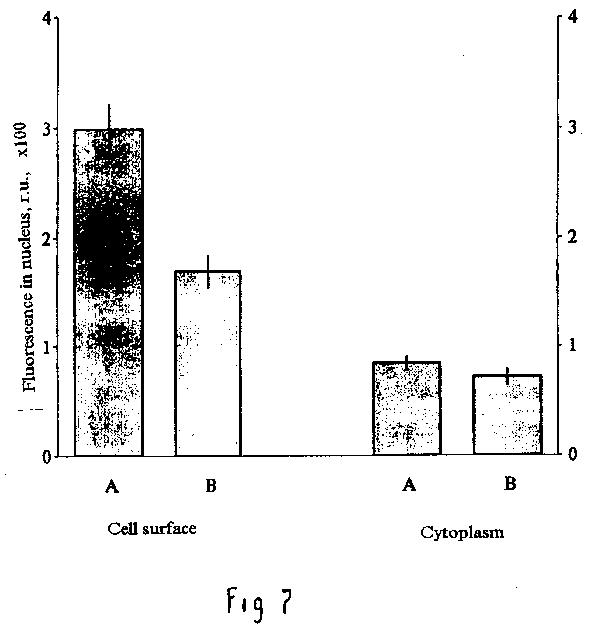

The binding of soluble complexes of a carrier and a genetic material was observed by confocal laser scanning microscopy. First, the conjugate carrier was labeled fluorosceinisocyanate. 7 minutes after the addition of the complexes at concentration 3.7 nM to PLC/PRF/5 cells ( at 21^{°} C), there were observed intensive fluorescent sites on cell surface. At the presence of 10 µ 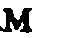 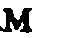 of free insulin, the average fluorescence intensity of these fluorescent sites on cell surface was considerably less as compared with that, when cells were incubated with FITC-labeled complexes only, as shown in Fig. 7, where A - cells after incubation with the complex; B- cells after incubation with a soluble complex and excess ( 10 µc ) free insulin. At the same time, there was observe no significant differences in the cytoplasm fluorescence of cells incubated with soluble complexes with or without excess of free insulin ( Fig. 7), that is under given conditions receptor-mediated endocytosis was not yet observed.

### EXAMPLE 4

### Receptor-mediated endocytosis of a soluble carrier-genetic material complex

To study receptor - mediated endocytosis, PLC/PFR/5 cells (after adsorption of the fluorescein-labeled insulin-polylisine-plasmid pSVRP8 construct) were examined by fluorescence image microscopy. Insulinpolylysin was first labeled with fluoresceinisothiocyanate.

PLC/PRF/5 cells were incubated with 7 nM a soluble fluorescence - labeled of insulin-polylisine-plasmid complex for 2 hours at 37^{o}C. Immediately ahead of the recording ( 10 minutes ), ammonium chloride ( at the final concentration of 10 mM ) was added, and fluorescent cell images were obtained by image microscopy, which were collected and processed at image analyzer.

The results of the study show that the FITC-labelled complex was accumulated in cells: the cytoplasm fluorescence intensity after incubation of cells with the FITC-labeled complex was considerably higher than that, when cells were incubated with the complexes containing excess free insulin, or cells incubated without using such complex ( autofluorescence ).

The frequency of distribution of the fluorescence intensity in cell cytoplasm also confirms this assumptions. In cells incubated with the FITC-labeled complexes at the presence of excess of free insulin, there were also observed different cytoplasm bands with higher fluorescence intensity than in case of autofluorescence. However, the intensity fluorescence of these more intensive fluorescent bands is lower considerably, as compared with that of cells incubated such complexes only.

In order to obtain comprehensive date and quantitative characteristics concerning the cell localization, such insulin-polylisine-plasmid complexes of PLC/PRF/5 cells were examined by confocal scanning laser microscopy.

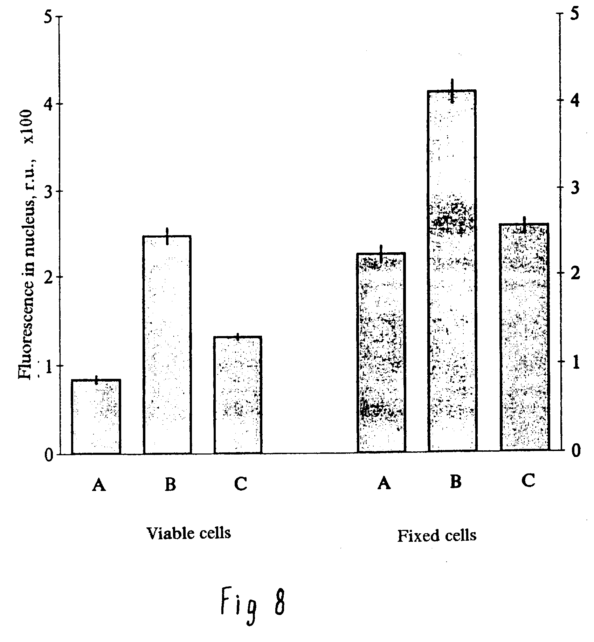

In the internalization studies, cells were incubated with 3,7 nM fluorescence-labeled complexes at 37^{o}C. After a 2 h incubation of cells under these conditions, there was observed the specific (free insulin-blocked ) accumulation of FITC-labelled insulin-polylisine - plasmid complexes in cell nuclei, as shown in Fig.8 where A-autofluorescence (control ); B- cells after incubation with complex; C - cell after incubation with a soluble complex and excess ( 10µM ) of free insulin; fixed cells - cells treated 4%- formaldehyde solution after incubation . Flurorescence-labeled complexes were accumulated in perinuclear sites where the fluorecsence intensity was almost three times higher than that of the cell autofluorescence, that was the evidence of the endocytosis of carrier-genetic material complexes. The fluorescence intensity in periniclear sites was 1,6 higher for cells incubated with the complexes without (1703 ±165 r.u.) than with the excess of free insulin ( 1062 ± 46 r.u, significant difference p< 0.001. From the above data it may be concluded that the soluble carrier-genetic material complexes can be uptaken by cells through the specific receptor-mediated endocytosis.

The addition ammonium chloride to the culture medium at the concentration of 10 mM 10 minutes before measurements accomplished by scanning laser microscopy resulted in increasing markedly the fluorescence of the endocytised complexes. This was strong proof that the complexes, after absorption, were brought into acid compartments of cells. The addition of the excess of free insulin caused a considerable decrease the fluorescence intensity, as measured under identical conditions (Fig. 8, viable cells ). These observations were comparable with the results obtained in case of the preparations with formaldehyde-treated cells ( Fig. 8, fixed cells ).

From the obtained results it could be concluded that the FITC-labelled insulin-polylisine-plasmid DNA complexes entered the PLC/PRF/5 cells by receptor- mediated endocytosis through the insulin receptors and, as a consequence, were transported specifically to the acid intracellular compartments.

### EXAMPLE 5

### Cytoplasm-nuclear transport of carrier

The capacity of the synthesized, insulin-polylysine conjugate carrier, to provide the cytoplasm-nuclear transport was examined by the fluorescence image restoration method after photobleaching. This method provides the quantitative characterization of the transport of the fluorescence-labeled molecules to the nuclei of viable cells. Insulin-polylysine carrier was first labeled with fluoresceinisothiocyanate. The PLC/PRF/5 cells were incubated with 4 mM of the fluorescence-of labeled insulin - polylysine conjugate for 4 hours and then were examined using the apparatus for measuring the fluorescence restoration after photobleaching.

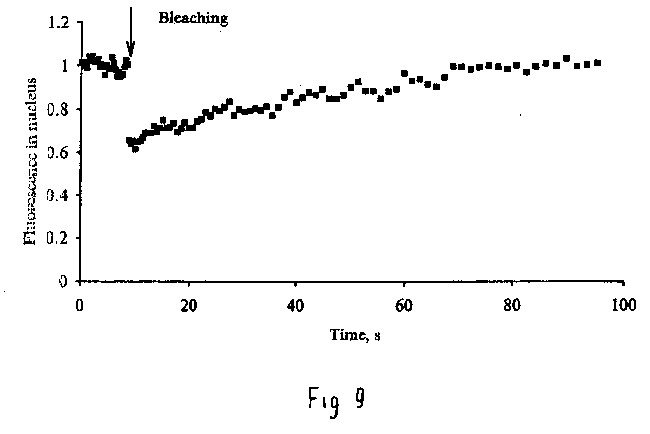

Fig. 9 shows the kinetics of the transport of FITC-labeled insulin-polylysine conjugate to the cell nuclei The results of the measurements showed that insulin-polylysine conjugate carrier was capable of providing the transport to target cell nuclei. The results of the studies performed by the Peters method ( Peters R. Nuclear envelope permeability measured by Fluorescence microphotolysis of single liver cell nuclei. J.Biol.Chem., v.258,p. 11427-11429 (1983) ) made it possible to determine the constant for the transport of the carrier to cell nuclei, which was of 1 0.035 ± 0.006 sec⁻¹. Thus, the synthesized carrier had the capacity to penetrate into cell nuclei through of the cytoplasm-nuclear transport mechanism.

### EXAMPLE 6

### The targeted delivery of soluble carrier-genetic material complexes (insulin-polylisine-plasmid) to cell nuclei

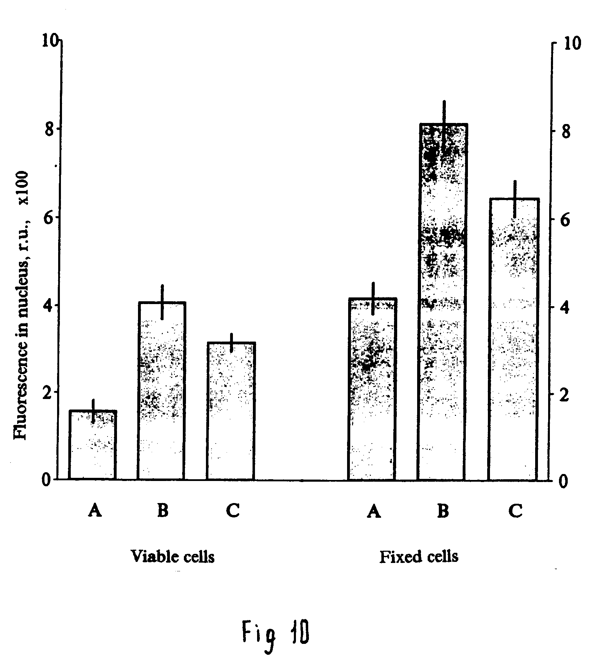

The results of the studies of soluble complex carrier - genetic material (flurorescence-labeled insulinpolylisine-plasmid DNA) conducted by confocal scanning laser microscopy using PLC/PRF/5 cells showed the presence of complexes in cell nuclei, as seen in Fig. 10 where A- autofluorescence ( control ); B- cells after incubation with such complex; C - cells after incubation with the soluble complex and the excess (5 µM) of free insulin; fixed cells - cells treated with by 4 % formaldehyde solution after incubation.

After a 2 h incubation of cells at 37^{o}C with 3,7 nM of soluble insulin-polylisine-plasmid pSVRP8 complex, there was observed the significant increase of the fluorescence intensity of the FITC-labeled complex in cell nuclei ( Fig. 10, viable cells). The identical results were obtained under studies of the preparations with fixed cells (Fig.10). In cells incubated with a soluble complex containing the excess of free insulin, there was smaller accumulation of the fluorescence-labeled complex in cell nuclei. After a 4- h incubation, there was observed the increase of fluorescence intensity of the complexes in cell nuclei.

### EXAMPLE 7

### The test for possibility of genetic transformation of cells with soluble carrier - genetic material complexes (insulin-polylisine-plasmid)

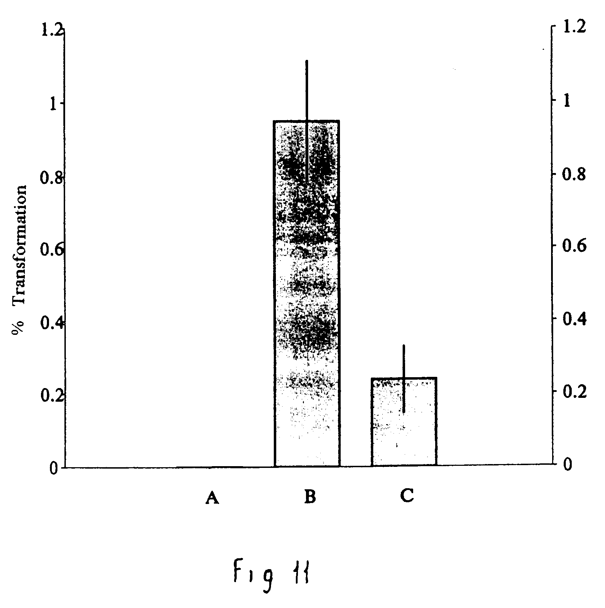

PLC/PRF/5 cells ( purified from serum) were incubated for 18 hours at 37^{o}C with soluble complexes of insulin-polylysine-plasmid pSVRP8. After the fixation and staining of cells by indirect immunofluorescence assay (the primary antibodies against super SV-40 T-antigen and the secondary fluorescence-labeled antibodies ), there were observed the presence of the cells with bright fluorescent nuclei ( as the gene expression producer being an envelope protein ), that was the evidence of high level gene expression of super T- antigen in such cells. These T- antigen-expressing cells were not found at all among the control with dead fluorescence nuclei, as shown in Fig. 11, where A- plasmid free of carrier ( Control), B - insulin-polylysine-plasmid complex (0.1 nM), C- insulin-polylisine-plasmid complex with the 400- fold excess of free insulin. The expression efficiency in case of incubating the hepatome cells with such construct was 0.95±0.16% ( ± - the mean standard error for the three representative experiment series where about 15000 cells were counted - Fig. 11 B). Free insulin added to cells together with soluble complexes decreased significantly the count of T antigen-expressing cells up to 0.24±0.09% (p< 0.001), as compared with that of the cells incubated with the construct only. This 75 % decrease of gene expression efficiency with adding the excess of free insulin on 75 % was consistent with residual binding ( 10-30 % ) which was observable under studies for the binding of ¹²⁵I-labeled insulin with cell PLC/PRF/5 line under identical conditions. In cell incubated with plasmid pSVRP8 without addition of the carrier, there was observed no super T-antigen expression.( Fig. 11, A).

### EXAMPLE 8

### The test of possibility of genetic transformation of mammary secretory cells using soluble carrier-genetic material complexes ( insulin-polylisine-plasmid )

The mouse mamary epithelial secretory cell line HC-11 was transformed using DNA complexes with carries ( insulin-polylisine-plasmid pRSVL) . This plasmid includes Lampyrida luciferase gene the expression of which could easily be recognized in cells. HC-11 cells had, according to our results, about 3000 insulin receptors per cell and the dissociation constant of the insulin receptor complex of about 4 nM, that made the cells suited for their modification using a soluble carrier- genetic material complex.

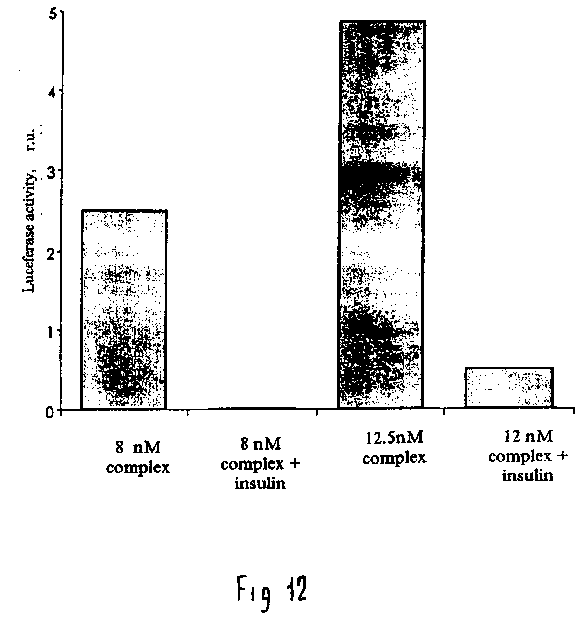

As the carrier, there was used insulin-polylysine conjugate synthesized as described in Example 1. The cells were planted on 6-cm Petri dishes ( Costar ) in RPMI-1640 medium supplemented with 10% fetal calf serum and incubated for 2- days at 37 ^{o}C and 5% Co_{2.} The incubated cells were washed from the medium and incubated for 3 hours in the RPMI-1640 medium without using the serum. To cells, there was added 0.5 ml of the soluble carrier-genetic material complex prepared as described in Example 2 in RPMI-1640 medium supplemented with 25 mM HEPES, 2 mg/ml BSA, pH 7,4. The cells were incubated with such complexes for 18 hours at 37^{o}C. Thereafter, there was added 5 ml of RPMI-1640 medium supplemented with 5 % fetal calf serum.1 day later, In a day, the grown cells were harvested, and the luciferase activity was measured in cell extracts using luminometer Pharmacia-LRB,model 1250. The results of experiments showed that mammary secretory cells could be modified genetically using the soluble carrier-genetic material complexes , as shown in Fig.12.

With the excess of free insulin ( 1.6 µM ) added to the cells together with the soluble complexes, the genetic transformation efficiency was about 1 order lower (Fig. 12).

Thus, it may be safely suggested that mammary cells, grown in culture, could be modified specifically with using the carrier - genetic material complex.

### EXAMPLE 9

### The specific genetic transformation of mammary gland in vivo

In order to test the possibility of transferring the Lampyrida luciferase gene into mammary cells in vivo and producing the protein producer of such gene in cells, a soluble carrier - genetic material complex comprising the insulin- polylysine conjugate at insulin/polylysine ratio of about 8:1, and Lampyrida luciferase-bearing plasmid pRVSL was injected into mammary ducts using the device of the present invention. The carrier was synthesized described in Example 1, and the soluble complex was prepared according to Example 2.

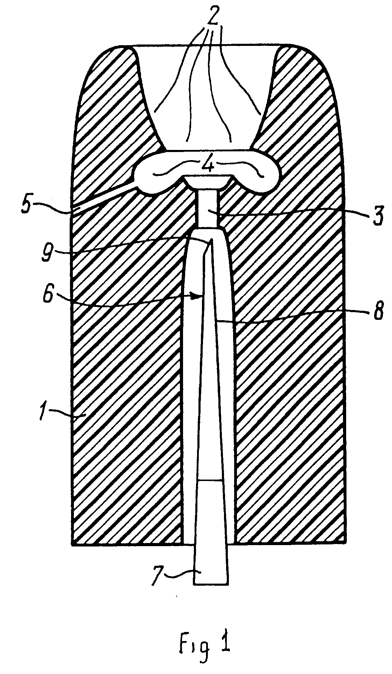

The solution containing such complex ( at concentration 37.5 nM ) was placed in the Hanks' balanced salt solution of the following composition: NaCl ( 0.137 mM ), KCl ( 5.36 mM ), CaCl₂ ( 1.26 mM ), MgSO₄ x7 H₂ O ( 0.41 mM ), MgCl₂ x 6 H20 ( 0.49 mM ), Na₂ HPO₄ x7 H₂ O ( 0.34 mM ), KH₂ PO₄ ( 0.44 mM ), pH 7.6, glucose (1.00 g/l) without using phenol red. 25 µl of the the resulting solution was injected into mammary duct of female mice BALB using the device of the invention ( Fig. 1).

The device was first charged with Hanks' balanced salt solution so that the recess with annular groove and the central opening were filled, and after sucking the device animal body, the transport means filled with the soluble carrier-genetic material was introduced through the filling solution into the mammary duct.

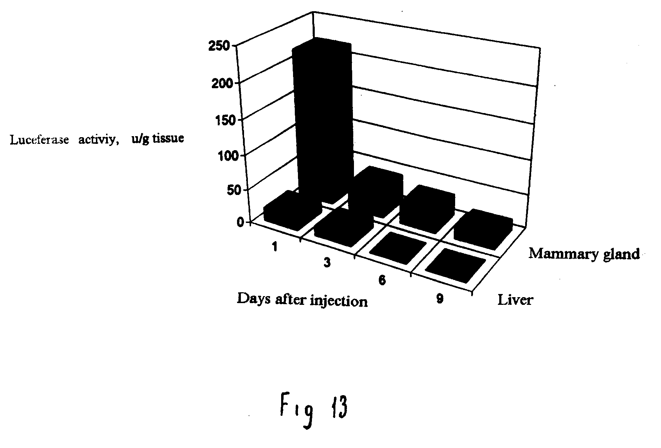

The results of injecting the construct lended support to the view that luciferase gene could be transferred using the chosen construct according to the method of the invention. This was evidenced by the presence of the luciferase activity in the mice mammary homogenate preparations, as it is shown on Fig. 13, which was measured by luminometer. In mice mamary tissue of the control, there was found no the luciferase activity. The highest activity of the marker luciferase enzyme was observed in a day after injection, and 9 days later, the significant content of luciferase activity in tissues was still detectable( Fig. 13).

With introducing the complex of genetic material with carrier into the part of the mice mammary glands, there was detectable no luciferase activity in other ( non-transformed ) mammary glands. In liver, the main target - organ for insulin, there were detectable the traces of luciferase activity only.

Thus, the possibility of site-specific delivery of a genetic material into animal mammary glands was demonstrated.

### EXAMPLE 10

### The confirmation test for the traumatical security and safety of the device for the injection of genetic material in animal mammary duct

The possibility of injuries under introduction of the transport means into mammary duct was tested on laboratory animals, mice BALB by injecting 0,4 % solution of trypan blue having molecular 960.8 at the amount of 100µ 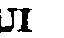 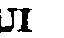 into each duct of the mammary gland using the special samples manufactured on microforge of tips having needle with fused (heat-polished ) edges.

The results of microinjections were examined under microscope Biolam with lens 2.5 x and 10 x and eye-pieces 15 x using mamary tissue preparations. None of the 90 mammary tissue preparations under test was damaged.

While the invention has been shown as the preferred embodiment, it will be obvious to those skilled in the art that it is not so limited, but is susceptible of various changes and modifications within the scope of the claims. Thus, for example, as an alternative of using one channel for communicating with the vacuum source, there may offer a set of such channels being equally arranged across the outer side of a annular groove.

Thus, the device of the present invention provides the safe and traumatically secure microinjection of a genetic material into animal mammary duct

### THE INDUSTRIAL APPLICABILITY

The method and the device of the present invention are applicable for experimental medicine, veterinary and pedigree stock-rainsing. During the manufacture of the device disclosed, there were used biologically inert plastic materials and the known methods for injection molding.

## Claims

1. A method for the specific genetic transformation of animal mammary gland comprising the injection of a genetic material into animal body in soluble complex with carriers which deliver said genetic material to target cell by specific receptor-mediated endocytosis, wherein said genetic material is further introduced into cell nuclei by cytoplasm-nuclear transport aided with carriers which deliver said genetic material directly to target cell nuclei, with said soluble complex of said genetic material with carriers injected into animal body cell or cell nuclei through mammary duct.

2. A method for the genetic transformation according to claim 1 wherein the genetic material is a nucleic acid.

3. A method for the genetic transformation according to claim 2 wherein the nucleic acid is DNA.

4. A method for the genetic transformation according to claim 1 wherein the carrier is said polyatomic cation(s)-ligand(s) conjugate.

5. A method for the genetic transformation according to claim 4 wherein the conjugate is covalently bound to the ligand.

6. A method for the genetic transformation according to claim 5 wherein the polyatomic cation is polylysine.

7. A method for the genetic transformation according to claim 4 wherein the ligand is insulin.

8. A method for the genetic transformation according to claim 1 wherein the carrier is the conjugate of polylysine covalently bound to insulin which is prepared by the addition of insulin to polylisine by modifying the terminal ends of insulin with citraconic anhydride, modifying the polylysine and insulin with cuccinimidylpyridinedithiopropionate, reducing the pyridindithionylated polylysine, dissolving both components in 2 M guanidine and modifying the pyridindithionylated insulin with freshly reduced SH-polylisine with subsequent 10% modification of amino groups by titration under stirring and incubation of the product.

9. A device for injecting a genetic material into animal mammary duct, wherein said device is the known teat cup per se, having case (1) with recess (2) to place the teat of a mammary gland with tightly- fitting therein, and provided with central opening (3) at the bottom of the recess, with the recess bottom having annular groove (4) surrounding the central opening (3) and communicating with vacuum source (5) to dilate the teat sphincter, and central opening ( 3) providing the entry of transport means (6) for the delivery of said genetic material into mammary duct.

10. A device according to claims 9, wherein case 1 is made from a plastic material and provided with the means to change the working volume and the shape of recess (2).

11. A device according to claims 9-10, wherein transport means 6 for the delivery a said genetic material is a conventional cannula per se, having measuring capacity (7) for said genetic material, and hollow tip (8 ) with the outer diameter corresponding the diameter of central opening (3) for supply the material to mammary duct.

12. A device according to claims 9-11, wherein measuring capacity (7) is made from a plastic material and serves as the vessel for said genetic material and the means to inject the material into tip (8).

13. A device according to claims 9-12, wherein tip (8) has needle (9) being tapered at 30-45^{°} longitudinally to tip( 8), and the edges of tip ( 9) are fused.

14. A device according claims 9-13, wherein the outer side of tip (8) is conical shape.

15. A device according to claims 9-14, wherein said device has a plurality of channels (5) located peripherally around recess (4) to provide the communication of annular groove (4) with the vacuum source.
